(19) Europäisches Patentamt
European Patent Office
Office européen des brevets

(11) **EP 1 860 999 B1**

(12) **EUROPÄISCHE PATENTSCHRIFT**

(45) Veröffentlichungstag und Bekanntmachung des Hinweises auf die Patenterteilung:
**23.12.2009 Patentblatt 2009/52**

(51) Int Cl.:
***A61B 5/0205*** *(2006.01)* ***A61B 5/0285*** *(2006.01)*

(21) Anmeldenummer: 06723463.3

(22) Anmeldetag: **16.03.2006**

(86) Internationale Anmeldenummer:
**PCT/EP2006/002410**

(87) Internationale Veröffentlichungsnummer:
**WO 2006/099988 (28.09.2006 Gazette 2006/39)**

(54) **MOBILES DIAGNOSEGERÄT**

MOBILE DIAGNOSIS DEVICE

DISPOSITIF DE DIAGNOSTIC MOBILE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.03.2005 DE 102005013429**

(43) Veröffentlichungstag der Anmeldung:
**05.12.2007 Patentblatt 2007/49**

(73) Patentinhaber: **Flore, Ingo**
**44141 Dortmund (DE)**

(72) Erfinder:
• **CHO, Ok Kyung**
**58239 Schwerte (DE)**

• **KIM, Yoon Ok**
**58239 Schwerte (DE)**

(74) Vertreter: **Isfort, Olaf**
**Schneiders & Behrendt,**
**Huestrasse 23**
**44787 Bochum (DE)**

(56) Entgegenhaltungen:
**EP-A- 1 317 902      US-A- 4 934 372**
**US-A- 5 309 916      US-A- 2003 109 901**
**US-A1- 2001 012 916      US-A1- 2002 087 087**
**US-A1- 2004 116 784      US-A1- 2004 162 493**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein mobiles Diagnosegerät nach dem Oberbegriff von Patentanspruch 1.

Herz-Kreislauf-Erkrankungen sind bekanntlich in fast allen Industrieländern die Haupttodesursache. Einen besonderen Stellenwert hat hierbei die Arteriosklerose, d. h. die krankhafte Verengung von Blutgefäßen. Bei ca. 50 % der an Arteriosklerose erkrankten Patienten liegt auch eine koronare Herzkrankheit vor. Aufgrund der deutlich zu erkennenden Progression von Herz-Kreislauf-Erkrankungen und der eingeschränkten therapeutischen Möglichkeiten in den Spätstadien dieser Erkrankungen wird eine möglichst frühzeitige Diagnose angestrebt. Hierzu ist es erforderlich, komplexe Zusammenhänge im Herz-Kreislauf-System zu erfassen und auszuwerten. Sowohl das Herz als auch die Blutgefäße müssen für eine frühzeitige Diagnose gleichermaßen in ihrem Funktionszustand bewertet werden. Ein gesundes Gefäßsystem kann leichtere Insuffizienzen des Herzens bekanntlich über Jahre ausgleichen, während allerdings ein bereits an Arteriosklerose erkranktes Gefäßsystem der Kreislaufdekompensation Vorschub leistet.

Das EKG (Elektrokardiogramm) dürfte die am meisten eingesetzte Untersuchungsmodalität zur Diagnose von Herz-Kreislauf-Erkrankungen sein. Mittels eines EKG-Gerätes werden mit zwei oder mehr EKG-Elektroden elektrische Signale von dem Körper des zu untersuchenden Patienten abgeleitet. Das so gewonnene EKG gibt die bioelektrischen Spannungen, die bei der Erregungsausbreitung und -rückbildung am Herzen entstehen, wieder. Das EKG enthält zahlreiche diagnostisch auswertbare Parameter. Zum Zeitpunkt der Kontraktion des Herzmuskels während eines Herzschlags zeigt das EKG eine deutliche Spitze, die auch als R-Zacke bezeichnet wird. Weiterhin enthält das EKG die der R-Zacke vorangehende, so genannte P-Welle. Der R-Zacke folgt wiederum die so genannte T-Welle. Die Minima im EKG unmittelbar vor und unmittelbar nach der R-Zacke werden mit Q bzw. S bezeichnet. Für die Herz-Kreislauf-Diagnostik interessante Parameter sind die Dauer der P-Welle sowie die Amplitude der P-Welle, die Dauer des PQ-Intervalls, die Dauer des QRS-Komplexes, die Dauer des QT-Intervalls sowie die Amplitude der T-Welle. Sowohl aus den Absolutwerten der genannten Parameter wie auch aus den Verhältnissen der Parameter kann auf den Gesundheitszustand des Herz-Kreislauf-Systems geschlossen werden.

Mittels der so genannten Plethysmographie ist die Erfassung peripherer Herz-Kreislauf-Parameter möglich. Bei der Plethysmographie werden durchblutungsbedingte Volumenschwankungen eines peripheren Blutgefäßes gemessen. Heutzutage hat sich die NIRP-Methode (engl. "near infrared photo plethysmography") durchgesetzt. Die dabei verwendeten Diagnosemodalitäten werden auch kurz als Pulsoximeter bezeichnet. Derartige Pulsoximeter umfassen typischerweise zwei Lichtquellen, die rotes bzw. infrarotes Licht unterschiedlicher Wellenlänge in das Körpergewebe des Patienten einstrahlen. Das Licht wird im Körpergewebe des Patienten gestreut und teilweise absorbiert. Das gestreute Licht wird mittels eines Lichtsensors in Form einer geeigneten Photozelle detektiert. Typischerweise verwenden kommerzielle Pulsoximeter zum einen Licht im Wellenlängenbereich von 660 nm. In diesem Bereich ist die Lichtabsorption von Oxyhämoglobin und Desoxyhämoglobin stark unterschiedlich. Dementsprechend variiert die Intensität des mittels des Lichtsensors detektierten, gestreuten Lichts in Abhängigkeit davon, wie stark das untersuchte Körpergewebe von sauerstoffreichem bzw. sauerstoffarmem Blut durchblutet ist. Zum anderen wird üblicherweise Licht im Wellenlängenbereich von 810 nm verwendet. Diese Lichtwellenlänge liegt im so genannten nahen infraroten Spektralbereich. Die Lichtabsorption von Oxyhämoglobin und Desoxyhämoglobin ist in diesem Spektralbereich im Wesentlichen gleich. Die bekannten Pulsoximeter sind in der Lage, ein Volumenpuls-Signal zu erzeugen, das die während des Herzschlags veränderliche Blutmenge wiedergibt, die das von dem Pulsoximeter erfasste Mikrogefäßsystem passiert. Bei Verwendung unterschiedlicher Lichtwellenlängen in den oben erwähnten Spektralbereichen kann aus der unterschiedlichen Lichtabsorption auf den Sauerstoffgehalt des Blutes (Sauerstoffsättigung) zurückgeschlossen werden. Die üblichen Pulsoximeter werden entweder an der Fingerspitze eines Patienten oder auch am Ohrläppchen eingesetzt. Es wird dann das Volumenpuls-Signal aus der Blutperfusion des Mikrogefäßsystems in diesen Bereichen des Körpergewebes erzeugt.

Aus der US 4,960,126 ist ein EKG-synchronisiertes Pulsoximeter vorbekannt. Das vorbekannte Gerät umfasst eine EKG-Einheit und eine Pulsoximetrie-Einheit. Die EKG-Einheit wird bei dem vorbekannten Diagnosegerät dazu genutzt, den Herzschlagzyklus durch Detektion von R-Zacken in dem EKG-Signal zu ermitteln. Die Dauer des Herzschlagzyklus wird dann bei der Bestimmung der Sauerstoffsättigung mittels der Pulsoximetrie-Einheit zugrunde gelegt. Dadurch sollen die Signalmittelung verbessert und bewegungsinduzierte Artefakte reduziert werden. Insgesamt wird eine zuverlässigere Bestimmung des Wertes der Sauerstoffsättigung des Blutes gegenüber herkömmlichen Pulsoximetern erreicht.

Die bekannten kombinierten EKG- und Pulsoximetrie-Geräte erlauben es, eine Vielzahl von Herz-Kreislauf-Parametern zu ermitteln. Anhand dieser Daten kann zwar ein behandelnder Arzt eine umfassende Herz-Kreislauf-Diagnostik durchführen. Die bekannten Geräte erlauben aber nachteiligerweise nicht die automatische Durchführung einer Vordiagnose von drohenden oder bereits bestehenden Herz-Kreislauf-Erkrankungen. Aus diesem Grund können die bekannten Geräte auch nicht ohne weiteres von Patienten zur Selbstdiagnose eingesetzt werden.

Die US 2002/0087087 A1 beschreibt ein Gerät zur Messung der arteriellen Pulswellengeschwindigkeit.

Das Gerät umfasst eine EKG-Einheit sowie einen Pulswellensensor. Die Pulswellengeschwindigkeit wird anhand der Zeitdifferenz zwischen einer R-Zacke im EKG-Signal und einem darauf folgenden Maximum des Signals des Pulswellensensors bestimmt.

Die US 5,309,916 beschreibt ein Gerät zur Messung einer Pulswellengeschwindigkeit anhand Haupt- und Nebenmaxima eines Puls-Signals.

Vor diesem Hintergrund ist es Aufgabe der Erfindung, eine verbesserte Zustands- und Trenddiagnostik des Herz-Kreislauf-Systems zu ermöglichen. Das Gerät soll dazu in der Lage sein, einem Patienten eine frühzeitige Selbstdiagnose einer Herz-Kreislauf-Erkrankung anzuzeigen, ohne dass der Patient mit der Auswertung einer Vielzahl von diagnostischen Parametern überfordert wird.

Diese Aufgabe löst die Erfindung durch ein mobiles Diagnosegerät nach Patentanspruch 1.

Die Erfindung basiert auf der Erkenntnis, dass die Kombination des EKG-Signals mit dem Volumenpuls-Signal bei einem kombinierten Diagnosegerät mit EKG-Einheit und Pulsoximetrie-Einheit eine einfache automatische Zustandsdiagnostik des Herz-Kreislauf-Systems ermöglicht. Mittels einer geeigneten Programmsteuerung ist die Auswertungseinheit des erfindungsgemäßen mobilen Diagnosegerätes dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund ihrer erfindungsgemäßen Programmsteuerung dazu in der Lage, Extrema, d.h. Minima oder Maxima, in dem Volumenpuls-Signal zu erkennen. Anhand der Extrema in dem Volumenpuls-Signal ist der Zeitpunkt des Eintreffens einer bei einem Herzschlag ausgelösten Pulswelle an dem von der Pulsoximetrie-Einheit erfassten peripheren Messort feststellbar. Somit kann schließlich der zeitliche Abstand zwischen einer R-Zacke in dem EKG-Signal und einem darauf folgenden Extremum in dem Volumenpuls-Signal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen abhängig. Aus der Elastizität der Blutgefäße kann wiederum auf eine ggf. vorliegende Arteriosklerose geschlossen werden. Weiterhin ist die Pulswellengeschwindigkeit vom Innendurchmesser der Arterlen abhängig. Dadurch ist es möglich, unter Annahme einer konstanten Elastizität und einer konstanten Dichte des Blutes die Durchblutung am jeweiligen Untersuchungsort (z.B. am Arm des Patienten) zu charakterisieren. Durch die Kombination des EKG-Signals mit dem Volumenpuls-Signal bei der automatischen Auswertung ist das erfindungsgemäße mobile Diagnosegerät zur funktionalen Bewertung des Gefäßsystems des Patienten selbsttätig in der Lage. Auf der Grundlage der automatisch ausgewerteten Signale kann das erfindungsgemäße Diagnosegerät den kardiovaskulären Zustand des Patienten grob einschätzen und bei Anzeichen einer Arteriosklerose ein entsprechendes Warnsignal für den Patienten erzeugen. Somit kann der Patient das erfindungsgemäße Diagnosegerät zur Selbstdiagnose verwenden. Es besteht nicht die Notwendigkeit der differenzierton Auswertung der von dem Gerät ermittelten verschiedenen Herz-Kreislauf-Parameter, die den Patienten in den meisten Fällen überfordern würde.

Gemäß einer sinnvollen Weiterbildung des erfindungsgemäßen Diagnosegerätes ist die Auswertungseinheit weiterhin eingerichtet

- zur Ermittlung der Blutsauerstoffsättigung aus dem Volumenpuls-Signal,

- zur Ermittlung der ventrikulären Herzfrequenz aus dem EKG-Signal,

- und/oder zur Ermittlung der plethysmographischen Herzfrequenz aus dem Volumenpuls-Signal.

Die Ermittlung der Blutsauerstoffsättigung, der ventrikulären und der plethysmographischen Herzfrequenz erlauben eine weitere und verfeinerte Zustandsdiagnostik des Herz-Kreislauf-Systems. Es können die Absolutwerte der Herzfrequenz, die Herzfrequenzvariabilität und entsprechende Arrhythmien des Herzens mit dem erfindungsgemäßen Diagnosegerät automatisch bestimmt werden. So können Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpuls-Signals feststellbar. Die Blutsauerstoffsättigung ist ebenfalls ein wichtiger Parameter bei der Zustandsdiagnostik des Herz-Kreislauf-Systems. Anhand der Blutsauerstoffsättigung kann auf die Leistungsfähigkeit und die Anpassungsfähigkeit des Herz-Kreislauf-Systems zurückgeschlossen werden.

Gemäß der Erfindung ist die automatische Erkennung von Haupt- und Nebenmaxima in dem Volumenpuls-Signal, die Ermittlung der Amplituden der Haupt- und Nebenmaxima und die Ermittlung des Zeitabstands zwischen den Haupt- und Nebenmaxima vorgesehen. Dies ermöglicht die automatische Untersuchung der Dikrotie des Volumenpuls-Signals. Mit Dikrotie bezeichnet man die Doppelgipfeligkeit des Blutdruckverlaufs in herzfernen Blutgefäßen. Die Dikrotie entsteht durch Überlagerung der vom Herzen ausgehenden Pulswelle mit einer rückläufigen Pulswelle aus der Reflexion der Pulswelle an Gefäßaufteilungen oder weniger elastischen herzfernen Gefäßabschnitten. Es ist bekannt, dass mit geringer werdender Elastizität und damit größer werdendem Grad einer Arteriosklerose der zeitliche Abstand zwischen den Haupt- und Nebenmaxima im plethysmographischen Signal geringer wird, wobei das Nebenmaximum gleichzeitig an Intensität verliert. Durch die Ermittlung des Zeitabstands zwischen Haupt- und Nebenmaxima und durch Bestimmung der relativen Amplituden der Haupt- und Nebenmaxima stehen somit weitere wichtige Parameter zur Verfügung, die mit dem erfindungsgemäßen Diagnosegerät genutzt werden können, um automatisch Anzeichen einer Arteriosklerose festzustellen.

Die Nebenmaxima in dem Volumenpuls-Signal entstehen vor allem durch Reflexion der Pulswellen an den unteren Extremitäten. Der zeitliche Abstand zwischen den Haupt- und Nebenmaxima wird daher im wesentlichen durch die Eigenschaften der Aorta bestimmt. Dies ermöglicht es, eine zweite Pulswellengeschwindigkeit zu bestimmen, nämlich die Pulswellengeschwindigkeit in der Aorta, während die erste Pulswellengeschwindigkeit, wie oben erläutert, am jeweiligen Messort (z.B. am Arm des Patienten) bestimmt wird. Mit dem erfindungsgemäßen Diagnosegerät können also mit Vorteil zwei verschiedene Pulswellengeschwindigkeiten bestimmt und zur Feststellung von Erkrankungen (einzeln oder kombiniert) ausgewertet werden.

Sinnvollerweise weist das erfindungsgemäße Diagnosegerät außerdem Sensoren zur Messung der Körpertemperatur des Patienten, der Umgebungstemperatur und/oder der Luftfeuchtigkeit auf. Diese Parameter sind insbesondere für die Kalibrierung der EKG-Einheit und der Pulsoximetrie-Einheit des erfindungsgemäßen Diagnosegerätes wichtig.

Eine besonders sinnvolle Ausgestaltung des erfindungsgemäßen Diagnosegerätes ergibt sich, wenn dieses eine Speichereinheit zur Speicherung der mittels der Auswertungseinheit bei einer Messung ermittelten Parameter unter gleichzeitiger Speicherung des Datums und/oder der Uhrzeit der Messung aufweist. Mittels der Speichereinheit können einerseits der Verlauf einer Erkrankung des Herz-Kreislauf-Systems und andererseits die Effekte einer entsprechenden Therapie verfolgt und dokumentiert werden. Andererseits können die in der Speichereinheit des Diagnosegerätes abgespeicherten Daten vom behandelnden Arzt ausgelesen und ausgewertet werden, um eine detaillierte Zustandsdiagnostik des Herz-Kreislauf-Systems durch den Arzt zu ermöglichen. Zweckmäßigerweise weist das erfindungsgemäße Diagnosegerät eine Datenübertragungsschnittstelle zur Übertragung der in der Speichereinheit des Diagnosegerätes gespeicherten Daten in einen Personalcomputer des Arztes auf. Hierbei kann es sich um eine übliche drahtgebundene oder auch um eine drahtlose Schnittstelle (beispielsweise nach dem Bluetooth-Standard) handeln.

Sinnvoll ist es weiterhin, wenn das erfindungsgemäße Diagnosegerät eine Diagnoseeinheit aufweist, die zur Bestimmung des Status des Herz-Kreislauf-Systems des Patienten aus den mittels der Auswertungseinheit ermittelten Parametern eingerichtet ist. Demgemäß hat das Diagnosegerät einen modularen Aufbau. Die Auswertungseinheit ist lediglich dafür zuständig, die erfassten Signale auszuwerten, um daraus die für die Diagnostik erforderlichen Parameter in der eben beschrieben Art und Weise zu bestimmen. Diese Parameter werden dann von der Diagnoseeinheit des Diagnosegerätes weiter verarbeitet, um daraus Rückschlüsse hinsichtlich des Status des Herz-Kreislauf-Systems zu ziehen. Die Diagnoseeinheit ist auch dafür zuständig, das Vorliegen einer Arteriosklerose automatisch zu erkennen und ggf. ein entsprechendes Warnsignal für den Patienten zu erzeugen.

Vorteilhaft ist es, wenn die Diagnoseeinheit des erfindungsgemäßen Diagnosegerätes weiterhin dazu eingerichtet, aus der zeitabhängigen Veränderung der mittels der Speichereinheit gespeicherten Parameter Trends hinsichtlich der Veränderung des Status des Herz-Kreislauf-Systems des Patienten zu bestimmen. In einigen Fällen ist es nicht möglich, aus den mittels der Auswertungseinheit des Diagnosegerätes ermittelten Parametern direkt auf mögliche Erkrankungen zurückzuschließen. Eine Veränderung der Parameter, beispielsweise eine kontinuierliche Erhöhung der Pulswellengeschwindigkeit, kann jedoch auf eine sich entwickkelnde Herz-Kreislauf-Erkrankung im Frühstadium hindeuten. Derartige Trends können zur automatischen Erkennung von Erkrankungen genutzt werden, wenn das Diagnosegerät von einem Patienten über einen längeren Zeitraum wiederholt genutzt wird, wobei dann jeweils die mittels der Auswertungseinheit automatisch ermittelten Parameter mittels der Speichereinheit des Diagnosegerätes gespeichert werden.

Die Diagnoseeinheit des erfindungsgemäßen Diagnosegerätes kann zweckmäßigerweise eingerichtet sein zur Berechnung eines Elastizitätsparameters aus dem zeitlichen Abstand zwischen einer R-Zacke in dem EKG-Signal und einem darauf folgenden Extremum in dem Volumenpuls-Signal, wobei der Elastizitätsparameter ein Maß für die Elastizität der Blutgefäße des Patienten ist. Bekanntlich ist die Pulswellengeschwindigkeit (PWG) proportional zur Quadratwurzel aus dem Quotienten der Elastizität der Blutgefäße $\kappa$ und der Dichte des Blutes $\rho$. Es gilt die Formel

$$PWG = \sqrt{\kappa h / d\rho} \ .$$

Dabei ist κ die Elastizität der Blutgefäße, *h* die Wandstärke der Adern, *d* der Durchmesser der Gefäße und ρ die Dichte des Blutes. Aus diesem Zusammenhang läßt sich mittels der Diagnoseeinheit die Elastizität κ berechnen, wenn man die Dichte des Blutes ρ und die übrigen Parameter als konstant annimmt. Aus dem Elastizitätsparameter kann für sich genommen oder in Kombination mit weiteren der mit dem erfindungsgemäßen Diagnosegerät ermittelbaren Parameter des Herz-Kreislauf-Systems auf eine Arteriosklerose zurückgeschlossen werden.

Gemäß einer sinnvollen Ausgestaltung der Erfindung weist das Diagnosegerät eine Anzeigeeinheit zur Anzeige des EKG-Signals, des Volumenpuls-Signals und der mittels der Auswertungseinheit ermittelten Parameter auf. Der das Diagnosegerät benutzende Patient bzw. der behandelnde Arzt kann sämtliche Werte von der Anzeigeeinheit komfortabel ablesen. Gleichzeitig kann die ordnungsgemäße Funktion des Diagnosegerätes überprüft werden.

Gemäß der Erfindung sind die EKG-Einheit, die Pulsoximetrie-Einheit und die Auswertungseinheit in einem gemeinsamen Gehäuse untergebracht. Damit ist das Diagnosegerät kompakt aufgebaut und kann als mobiles Gerät jederzeit benutzt werden.

Die EKG-Elektroden sowie die Lichtquelle und der Lichtsensor der Pulsoximetrie-Einheit sind derart an der Außenseite des Gehäuses angeordnet, dass der Patient mit einer Hand eine erste EKG-Elektrode und mit der anderen Hand eine zweite EKG-Elektrode und gleichzeitig den Lichtsensor berühren kann. Das in dieser Weise ausgestaltete Diagnosegerät kann der Patient mit beiden Händen festhalten und gleichzeitig die Anzeige des Diagnosegerätes beobachten. Die EKG-Ableitung erfolgt dann von der linken und der rechten Hand des Patienten. Die Pulsoximetrie-Einheit erfasst gleichzeitig das Volumenpuls-Signal an einer der beiden Hände, die das Gehäuse berühren. Diese Ausgestaltung hat den Vorteil, dass keine zusätzlichen Elektroden über Kabelverbindungen an das Diagnosegerät angeschlossen werden müssen. Sämtliche Komponenten bilden eine kompakte Einheit. Fehler bei der Bedienung, insbesondere beim Anschluss der EKG-Elektroden und bei der Anbringung des Pulsoximeters am Körper des Patienten, sind ausgeschlossen. Ebenso kann das EKG-Signal über wenigstens zwei externe Elektroden aufgenommen werden, welche direkt auf den Körper des Patienten aufgeklebt werden können. Diese Anordnung hat den Vorteil, dass der Patient die Hände frei hat, z.B. um sich auf einem Ergometer festzuhalten oder abzustützen.

Das Diagnosegerät gemäß der Erfindung mit EKG-Einheit und Pulsoximetrie-Einheit kann durch eine geeignete Programmsteuerung der Auswertungseinheit eingesetzt werden. Ein Computerprogramm für die Auswertungseinheit eines solchen Diagnosegerätes umfasst Instruktionen

- zur automatischen Erkennung von R-Zacken in dem EKG-Signal,

- zur automatischen Erkennung von Extrema in dem Volumenpuls-Signal,

- und zur Ermittlung des zeitlichen Abstands zwischen einer R-Zacke in dem EKG-Signal und einem darauf folgenden Extremum in dem Volumenpuls-Signal.

Weiterhin umfasst das Computerprogramm Instruktionen zur Berechnung der Pulswellengeschwindigkeit aus dem zeitlichen Abstand zwischen einer R-Zacke in dem EKG-Signal und einem darauf folgenden Extremum in dem Volumenpuls-Signal. Die Pulswellengeschwindigkeit ist der zentrale Parameter, auf dem die Zustandsdiagnostik des Herz-Kreislauf-Systems mit dem erfindungsgemäßen Diagnosegerät wesentlich basiert.

Gemäß der Erfindung umfasst das Computerprogramm außerdem Instruktionen zur automatischen Erkennung von Haupt- und Nebenmaxima in dem Volumenpuls-Signal, zur Ermittlung des Zeitabstands zwischen Haupt- und Nebenmaxima und zur Berechnung einer zweiten Pulswellengeschwindigkeit aus dem zeitlichen Abstand zwischen Haupt- und Nebenmaxima. Diese zweite Pulswellengeschwindigkeit ist die Pulswellengeschwindigkeit in der Aorta.

Ein Computerprogramm der zuvor beschriebenen Art kann auch nicht im Sinne der Erfindung zur Auswertung eines EKG-Signals und eines Volumenpuls-Signals mittels eines üblichen PCs verwendet werden.

Ausführungsbeispiele der Erfindung werden im Folgenden unter Bezugnahme auf die Zeichnungen näher erläutert. Es zeigen:

Fig. 1        Darstellung des Aufbaus des erfindungs- gemäßen Diagnosegerätes anhand eine Blockdiagramms:

Fig. 2        Blockdiagramm-Darstellung der Pulsoxi- metrie-Einheit des erfindungsgemäßen Diagnosegerätes;

Fig. 3        Blockdiagramm-Darstellung der EKG-Ein- heit;

Fig. 4        Illustration der Ermittlung der Pulswellen- geschwindigkeit anhand eines Verfahrens- schemas;

Fig. 5 und 5a        Schaltschema der EKG-Einheit des erfindungsgemäßen Diagnosegerätes;

Fig. 6 und 6a        Schaltschema der Pulsoximetrie-Ein-

heit des erfindungsgemäßen Diagnosegerätes;

Fig. 7    Darstellung eines Volumenpuls-Signals zusammen mit einem EKG-Signal;

Fig. 8    Ansicht des erfindungsgemäßen Diagno- segerätes;

Fig. 9    Verfahrensschema zur Auswertung des EKG-Signals;

Fig. 10   Verfahrensschema zur Auswertung des Volumenpuls-Signals.

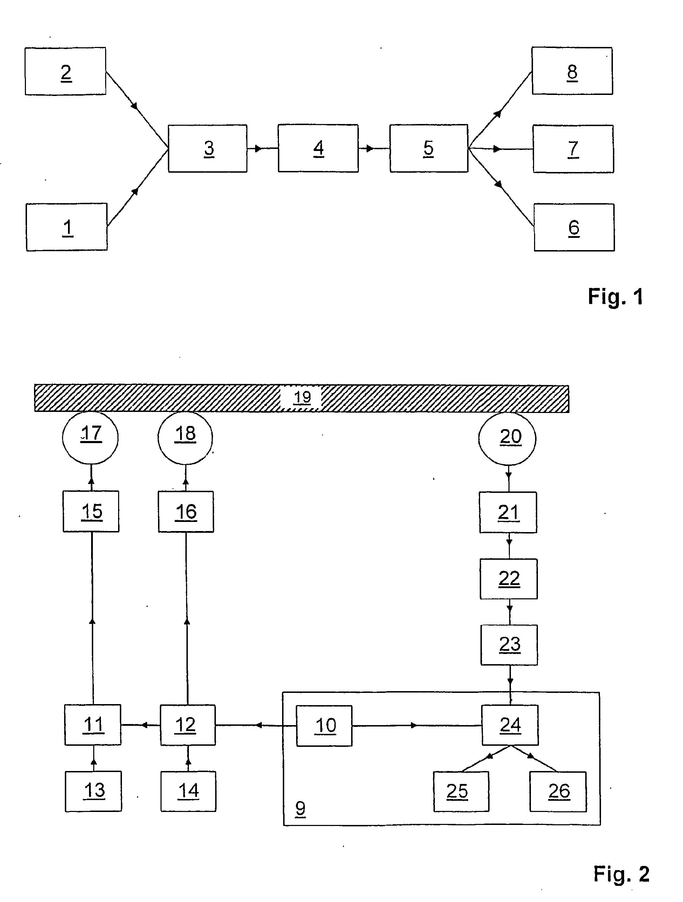

In der Fig. 1 sind die wesentlichen Komponenten des erfindungsgemäßen, mobilen Diagnosegerätes und deren Zusammenwirken veranschaulicht. Das Diagnosegerät umfasst eine EKG-Einheit 1 und eine Pulsoximetrie-Einheit 2. Die EKG-Einheit 1 ist mit in der Fig. 1 nicht näher dargestellten EKG-Elektroden zur Ableitung von elektrischen Signalen von dem Körper eines Patienten verbindbar. Die von der EKG-Einheit 1 erfassten EKG-Signale werden einer Analyse-Einheit 3 zugeführt. Die Pulsoximetrie-Einheit 2 dient zur optischen Messung der Blutperfusion im Mikrogefäßsystem des Körpergewebes des Patienten. Die von der Pulsoximetrie-Einheit 2 bei zwei verschiedenen Lichtwellenlängen erfassten Volumenpuls-Signale werden ebenfalls der Analyse-Einheit 3 zugeführt. Mittels der Analyse-Einheit 3 werden die Signale der Pulsoximetrie-Einheit 2 und der EKG-Einheit 1 vorverarbeitet. Insbesondere durchlaufen die Signale ein Bandpass-Filter, um Störungen im Bereich der Netzfrequenz von 50 bzw. 60 Hz herauszufiltern. Des Weiteren werden die Signale der Pulsoximetrie-Einheit 2 einer Mittelung unterzogen, um das Signalrauschen zu reduzieren. Nach Passieren der Analyse-Einheit 3 gelangen die aufbereiteten Signale der EKG-Einheit 1 und der Pulsoximetrie-Einheit 2 in eine Auswertungseinheit 4. Mittels der Auswertungseinheit 4 werden die zur Herz-Kreislauf-Diagnostik benötigten Parametern aus den Signalen extrahiert. Hierzu weist die Auswertungseinheit 4 eine entsprechende Programmsteuerung auf. Mittels dieser Programmsteuerung erfolgt eine automatische Erkennung von R-Zacken in dem EKG-Signal, eine automatische Erkennung von Extrema in dem Volumenpuls-Signal, und es wird der zeitliche Abstand zwischen einer R-Zacke in dem EKG-Signal und dem darauf folgenden Extremum, z.B. dem zeitlich nächsten Minimum, in dem Volumenpuls-Signal ermittelt. Weiterhin wird aus den Volumenpuls-Signalen der Pulsoximetrie-Einheit 2 die Blutsauerstoffsättigung ermittelt. Aus den Zeitabständen zwischen den R-Zacken in dem EKG-Signal wird die ventrikuläre Herzfrequenz bestimmt. Die plethysmographische Herzfrequenz wird aus den Volumenpuls-Signalen ermittelt. Weiterhin ist die Auswertungseinheit 4 durch ihre Programmsteuerung eingerichtet zur automatischen Erkennung von Haupt- und Nebenmaxima in den Volumenpuls-Signalen sowie zur Ermittlung der Amplituden der Haupt- und Nebenmaxima. Außerdem werden mittels der Auswertungseinheit 4 die Zeitabstände zwischen den Haupt- und Nebenmaxima in den Volumenpuls-Signalen bestimmt, woraus, wie oben erläutert, die Pulswellengeschwindigkeit in der Aorta bestimmbar ist. Die so durch die Auswertungseinheit 4 ermittelten Parameter werden einer Diagnoseeinheit 5 zugeführt. Die Diagnoseeinheit 5 ist zur Bestimmung des Status des Herz-Kreislauf-Systems aus den mittels der Auswertungseinheit 4 ermittelten Parametern eingerichtet. Mittels einer geeigneten Programmsteuerung interpretiert die Diagnoseeinheit 5 die relevanten Parameter, um daraus eine Einschätzung der Güte des Gefäßsystems des Patienten zu gewinnen und um festzustellen, ob die ermittelten Parameter auf eine bestehende Arterioskleroseerkrankung hinweisen. Die Diagnoseeinheit 5 wertet die Herzfrequenz aus, um festzustellen, ob eine Bradycardie oder eine Tachykardie vorliegt. Sonstige Irregularitäten des Herzschlags, wie z. B. Extrasystolen, können ebenfalls mittels der Diagnoseeinheit 5 festgestellt werden. Die Diagnoseeinheit 5 führt weiterhin einen Vergleich der ventrikulären Herzfrequenz mit der plethysmographischen Herzfrequenz durch, um ggf. Pulsdefizite festzustellen. Insbesondere ist aber die Diagnoseeinheit 5 durch ihre Programmsteuerung dazu eingerichtet, einen Elastizitätsparameter aus dem zeitlichen Abstand zwischen einer R-Zacke in dem EKG-Signal und einem darauf folgenden Extremum in dem Volumenpuls-Signal zu berechnen. Dabei ist der Elastizitätsparameter ein Maß für die Elastizität der Blutgefäße des Patienten. Durch Hinzunahme der weiteren mittels der Auswertungseinheit 4 ermittelten Parameter, insbesondere der Parameter hinsichtlich der Dikrotie des Volumenpuls-Signals, kann die Diagnoseeinheit 5 mit hoher Zuverlässigkeit selbsttätig den kardiovaskulären Zustand des Patienten analysieren. Dadurch ist das erfindungsgemäße Diagnosegerät für eine frühzeitige Diagnose einer koronaren Herzkrankheit hilfreich. Die mittels der Auswertungseinheit 4 ermittelten Parameter sowie die daraus mittels der Diagnoseeinheit 5 gewonnenen Daten werden schließlich in einer Speichereinheit 6 des erfindungsgemäßen Diagnosegerätes gespeichert, und zwar unter gleichzeitiger Speicherung des Datums und der Uhrzeit der jeweiligen Messung. Sämtliche gewonnenen Daten und Parameter sind mittels einer Anzeigeeinheit 7 anzeigbar. Insbesondere zeigt die Anzeigeeinheit 7 das EKG-Signal, das Volumenpuls-Signal und die Pulswellengeschwindigkeit an. Außerdem ist eine Schnittstelle 8 vorgesehen, die zur Verbindung des Diagnosegerätes mit einem Computer dient. Über die Schnittstelle 8 können sämtliche Daten und Parameter, insbesondere auch die in der Speichereinheit 6 gespeicherten Daten und Parameter, an einen nicht näher dargestellten PC des behandelnden Arztes übertragen werden. Dort können die Daten detaillierter analysiert werden. Insbesondere können die über einen längeren Zeitraum mit dem Diagnosegerät aufgenommenen Daten und Parameter auf Veränderungen hin untersucht werden, um daraus Trends hinsichtlich der Entwicklung einer bestehenden Erkrankung des Patienten ableiten zu können. Außerdem ermöglicht dies, den Erfolg einer Therapie detailliert nachzuverfolgen. Weiterhin ist es möglich, das erfindungsgemäße Diagnosegerät gleichsam als bloße Messdatenerfassungs- und -sendeeinheit zu benutzen und die aufgenommenen Signale direkt z.B. an den PC des Arztes zu übertragen, mittels welchem die entsprechenden Auswertungen, Berechnungen und Darstellungen (s.u.) dann (ggf. schneller und komfortabler) durchgeführt werden können.

Die Fig. 2 illustriert den Aufbau der Pulsoximetrie-Einheit 2 des erfindungsgemäßen Diagnosegerätes. Die Pulsoximetrie-Einheit 2 umfasst einen Mikrocontroller 9. Bestandteil des Mikrocontrollers 9 ist ein Timing-Generator 10, der einen Infrarotmodulator 11 und einen Rotmodulator 12 ansteuert. Mittels der Modulatoren 11 und 12 werden die Versorgungsspannungen von einstellbaren Spannungsversorgungen 13 und 14 moduliert. Die modulierten Spannungen werden über Strom/Spannungs-Konverter 15, 16 einer infrarotes Licht emittierenden Leuchtdiode 17 und einer rotes Licht emittierenden Leuchtdiode 18 zugeführt. Der Timing-Generator 10 sorgt dafür, dass die Leuchtdioden 17 und 18 abwechselnd ein- und ausgeschaltet werden. Somit wird das Körpergewebe 19 des Patienten abwechselnd mit rotem und mit infrarotem Licht bestrahlt. In dem Körpergewebe 19 wird das Licht gestreut und entsprechend dem Oxyhämoglobin- bzw. Desoxyhämoglobingehalt des Blutes, das das Gewebe 19 durchströmt, absorbiert. Das gestreute Licht wird von einem Photodetektor (einer Photodiode) 20 registriert. Der Photostrom des Photodetektors 20 wird mittels eines Konverters 21 in eine Spannung umgewandelt, mittels eines Verstärkers 22 verstärkt und mittels eines Analog/Digitalwandlers 23 in eine digitales Signal umgewandelt. Das Digitalsignal wird sodann einem Infrarot/Rot-Demodulator 24 zugeführt, der Bestandteil des Mikrocontrollers 9 ist. Der Infrarot/Rot-Demodulator 24 steht mit dem Timing-Generator 10 in Verbindung. Der Demodulator 24 trennt das Digitalsignal in zwei Volumenpuls-Signale 25 und 26 auf. Das Signal 25 gibt die Absorption des infraroten Lichtes in dem Gewebe 19 wieder, während das Signal 26 der Absorption des roten Lichtes in dem Gewebe 19 zugeordnet ist.

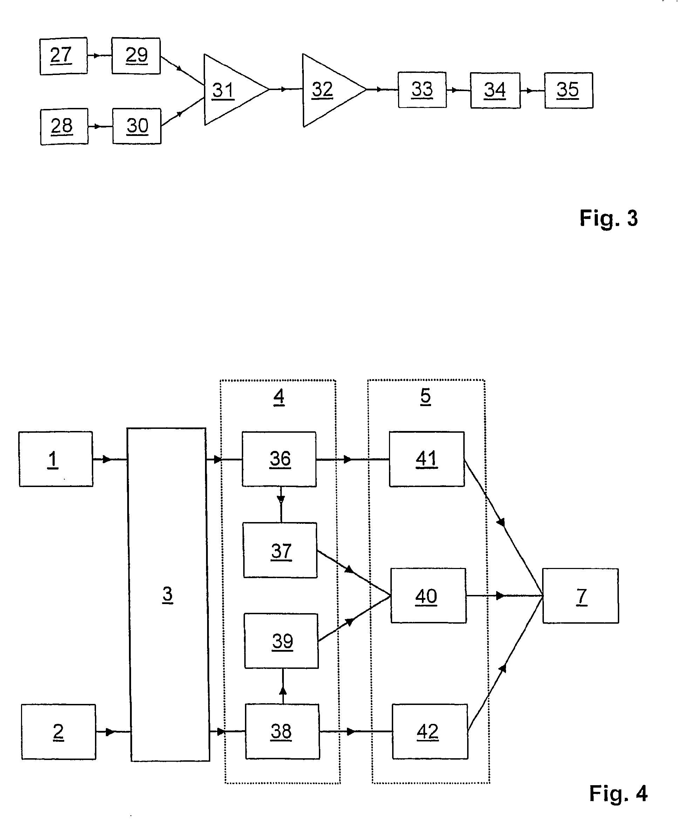

Anhand der Fig. 3 wird der Aufbau der EKG-Einheit 1 des erfindungsgemäßen Diagnosegerätes erläutert. An die EKG-Einheit 1 sind zwei EKG-Elektroden 27 und 28 angeschlossen. Die mittels der Elektroden 27 und 28 erfassten Signale durchlaufen zunächst Hochpass-Filter 29 und 30. Die Grenzfrequenz der Hochpass-Filter 29 und 30 liegt vorzugsweise zwischen 0,05 und 0,5 Hz. Die gefilterten Signale werden sodann einem Differenzverstärker 31 zugeführt. Dieser zeichnet sich durch eine hohe Gleichtaktunterdrückung aus, was zur Reduzierung von Bewegungsartefakten in dem EKG-Signal sinnvoll ist. Dem Differenzverstärker 31 folgt ein weiterer Verstärker 32 mit variablem Verstärkungsfaktor. Das so verstärkte Analogsignal wird mittels eines Analog/Digital-Wandlers 33 in ein Digitalsignal umgewandelt, das einem Mikrocontroller 34 (der mit dem Mikrocontroller 9 identisch sein kann) zugeführt wird. Schließlich wird eine Filterung 35 des digitalen Signals durchgeführt, um Störungen des Signals bei der Netzfrequenz von 50 bzw. 60 Hz aus dem EKG-Signal herauszufiltern.

Die Fig. 4 veranschaulicht die prinzipielle Funktionsweise des erfindungsgemäßen Diagnosegerätes. Die mittels der EKG-Einheit 1 und der Pulsoximetrie-Einheit 2 erfassten und mittels der Analyse-Einheit 3 aufbereiteten Signale werden mittels der Auswertungseinheit 4 ausgewertet, wie oben dargelegt. Die Auswertungseinheit 4 weist hierzu eine entsprechende Programmsteuerung auf, die zunächst in einem Verfahrensschritt 36 das EKG-Signal analysiert und die verschiedenen Zeitabstände des PQRST-Komplexes ermittelt. Insbesondere werden im Verfahrensschritt 36 die R-Zacken in dem EKG-Signal erkannt. Durch Zugriff auf eine in den Figuren nicht näher dargestellte Echtzeituhr des erfindungsgemäßen Diagnosegerätes werden in einem Verfahrensschritt 37 die genauen Zeitpunkte der detektierten R-Zacken ermittelt. Weiterhin umfasst die Programmsteuerung der Auswertungseinheit 4 eine Dikrotie-Berechnungsroutine 38. Diese ist dafür zuständig, in den digitalen Volumenpuls-Signalen automatisch Haupt- und Nebenmaxima zu erkennen, die Amplituden der Haupt- und Nebenmaxima sowie die Zeitabstände zwischen den Haupt- und Nebenmaxima zu ermitteln. Eine weitere Routine 39 bestimmt wiederum durch Zugriff auf die Echtzeituhr des Diagnosegerätes für jedes detektierte Hauptmaximum den genauen Zeitpunkt. Die Programmsteuerung der Diagnoseeinheit 5 enthält eine Routine 40 zur Berechnung eines Elastizitätsparameters aus dem zeitlichen Abstand zwischen den mittels der Routine 37 ermittelten Zeitpunkten der R-Zacken und den mittels der Routine 39 ermittelten Zeitpunkten der Hauptmaxima (oder auch der Minima) der Volumenpuls-Signale. Die Routine 40 ermittelt beispielsweise als Elastizitätsparameter die Pulswellengeschwindigkeit, die zum Zeitabstand zwischen einer R-Zacke in dem EKG-Signal und dem darauf folgenden Minimum in einem der Volumenpuls-Signale umgekehrt proportional ist. Der Elastizitätsparameter ist ein Maß für die Elastizität der Blutgefäße des Patienten und wird mittels der Anzeigeeinheit 7 angezeigt. Weiterhin umfasst die Diagnoseeinheit 5 eine Routine 41 zur Bewertung des ventrikulären Herzschlags aus dem digitalen EKG-Signal sowie eine Routine 42 zur Bewertung der Sauerstoffsättigung des Blutes aus den digitalen plethysmographischen Signalen. Die ventrikuläre Herzfrequenz sowie die Sauerstoffsättigung werden ebenfalls mittels der Anzeigeeinheit 7 angezeigt.

Die wichtigste Funktion des erfindungsgemäßen Diagnosegerätes ist, wie oben ausgeführt, die automatische, frühzeitige Erkennung einer Arterioskleroseerkrankung. Mit der Auswertungsroutine 38 der Auswertungseinheit 4 und der Diagnoseroutine 40 der Diagnoseeinheit 5 werden drei wichtige Parameter ermittelt, die charakteristisch für die Elastizität der Blutgefäße des Patienten sind. Anhand dieser drei Parameter kann daher auf eine bestehende Arteriosklerose und sogar auf die schwere einer bestehenden Erkrankung zurückgeschlossen werden. Diese drei Parameter sind der Zeitabstand zwischen Haupt- und Nebenmaxima in dem Volumenpuls-Signal, die relative Intensität der Haupt- und Nebenmaxima sowie die Pulswellengeschwindigkeit, die sich aus dem zeitlichen Abstand zwischen einer R-Zacke in dem EKG-Signal und dem darauf folgenden Extremum in dem Volumenpuls-Signal ergibt. Nach Auswertung dieser drei Parameter erzeugt das erfindungsgemäße Diagnosegerät ggf. ein Warnsignal, durch das dem Patienten z. B. der Rat gegeben wird, einen Arzt aufzusuchen. Der behandelnde Arzt kann dann die in der Speichereinheit 6 des Gerätes gespeicherten Daten detailliert auswerten und eine entsprechende Therapie für den Patienten festlegen.

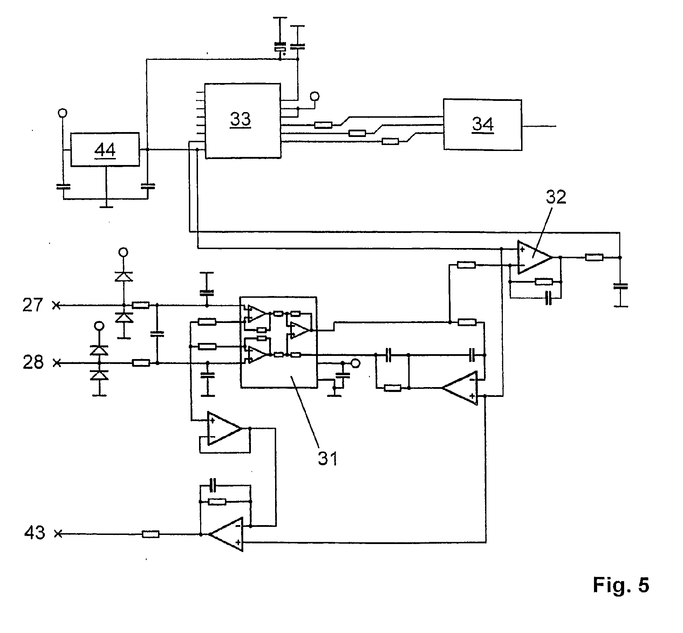

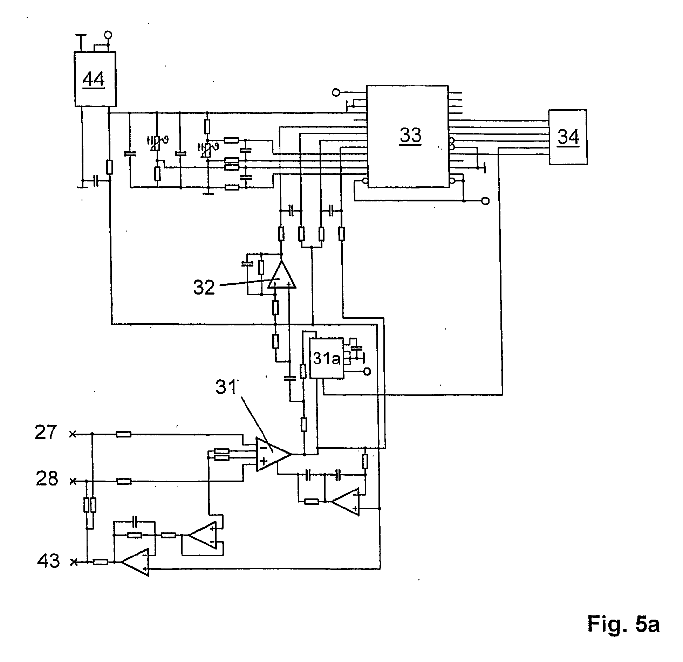

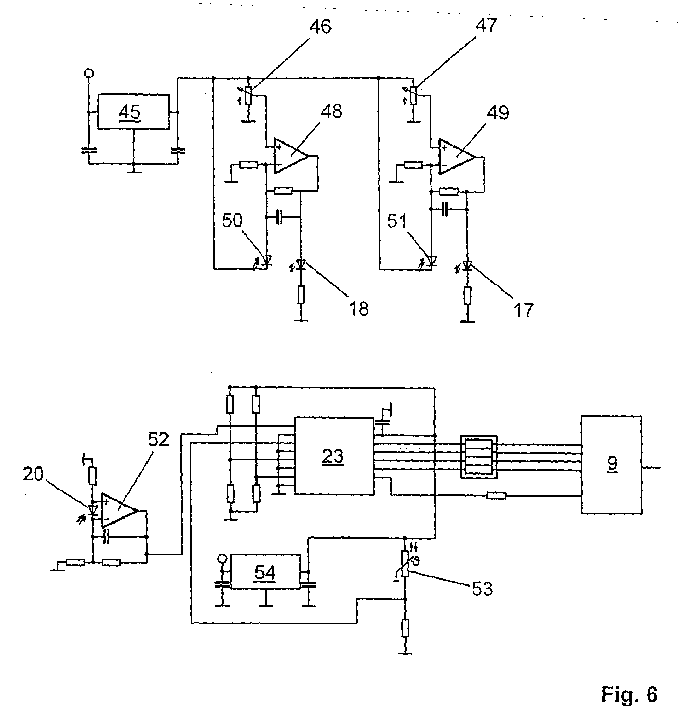

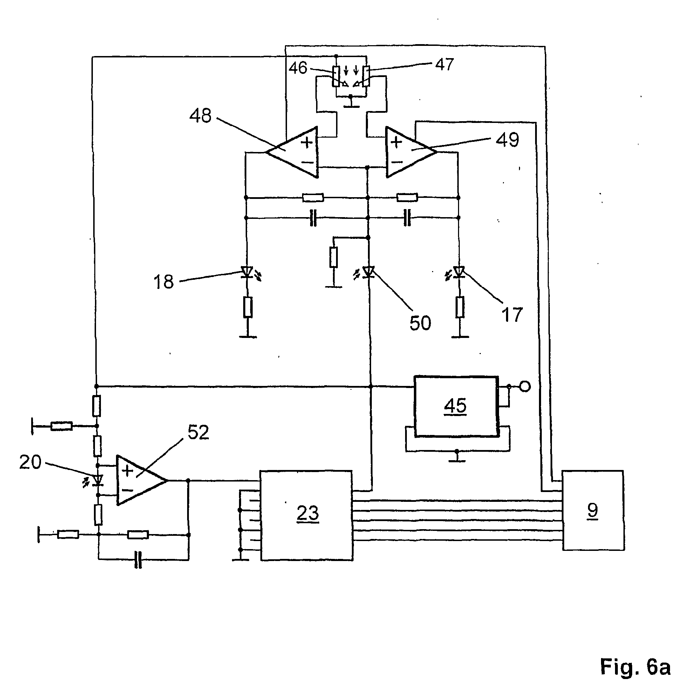

Das Schema gemäß Fig. 5 zeigt den prinzipiellen schaltungstechnischen Aufbau der EKG-Einheit 1 des erfindungsgemäßen Diagnosegerätes. Über die beiden EKG-Elektroden 27 und 28 werden elektrische Signale von dem Körper des Patienten abgeleitet. Diese elektrischen Signale werden zunächst mittels eines passiven Netzwerkes aus Dioden, Kondensatoren und Widerständen gefiltert. Danach werden die Signale dem Differenzverstärker 31 zugeführt. Dieser zeichnet sich durch eine hohe Gleichtaktunterdrückung aus. Dadurch werden an beiden Elektroden synchron anliegende Störungen aus dem EKG-Signal eliminiert. Über eine dritte Elektrode 43 kann ein invertiertes Gleichtaktsignal auf den Patienten zurückgeführt werden, wodurch die Signalsstörungen weiter reduziert werden. Das mittels eines variablen Verstärkers 32 verstärkte Analogsignal wird sodann mittels des Analog/Digital-Wandlers 33 in ein digitales EKG-Signal konvertiert, das dem Mikrocontroller 34 zugeführt wird. Der Analog/Digital-Wandler 33 steht mit einer Referenzspannungsquelle 44 in Verbindung. Gemäß Fig. 5a ist zusätzlich ein Notch-Filter 31a vorgesehen, um die Netzfrequenz (50 Hz bzw. 60 Hz) aus aus dem EKG-Signal herauszufiltern. Die Figuren 6 und 6a illustrieren den prinzipiellen schaltungstechnischen Aufbau der Pulsoximetrie-Einheit 2 des erfindungsgemäßen Diagnosegerätes. Die Verschaltung der infraroten LED 17 ist mit der Verschaltung der roten LED 18 identisch. Beide werden über eine Referenzspannungsquelle 45 mit Strom versorgt. Die Ansteuerung der Dioden 17 und 18 erfolgt über digitale Potentiometer 46 und 47. Diese werden von dem Mikrocontroller 9 der Pulsoximetrie-Einheit 2 angesteuert. Die Spannungen an den Ausgängen der Operationsverstärker 48 und 49 bestimmen zusammen mit den nachfolgenden Widerständen die Ströme, die durch die Leuchtdioden 17 bzw. 18 fließen. Photodioden 50 und 51 sind vorgesehen, die mit den Operationsverstärkern 48 und 49 verbunden sind. Die Schaltungsvariante gemäß Fig. 6a kommt mit nur einer Photodiode 50 aus. Hierdurch wird eine temperaturunabhängige, konstante Intensität des von den Dioden 17 und 18 emittierten Lichts geregelt. Das Licht der Leuchtdioden 17 und 18 wird mittels der Photodiode 20 detektiert. Die Photodiode 20 ist mit einem Operationsverstärker 52 verbunden, der den Strom durch die Photodiode 20 in eine Spannung umwandelt und verstärkt. Diese Spannung wird mittels des Analog/Digital-Wandlers 23 digitalisiert und nach einer Mittelung dem Mikrocontroller 9 der Pulsoximetrie-Einheit zugeführt. Weiterhin ist ein NTC-Widerstand 53 zur Messung der Körpertemperatur des Patienten am Messort der Pulsoximetrie-Einheit vorgesehen. Der NTC-Widerstand 53 ist mit dem Analog/Digital-Wandler 23 verbunden. Der NTC-Widerstand 53 ist Bestandteil eines einfachen Spannungsteilers, der über eine Referenzspannungsquelle 54 mit Spannung beaufschlagt wird. Die Referenzspannungsquelle 54 wird gleichzeitig für den Analog/Digital-Wandler 23 genutzt. Gemäß Fig. 6a ist nur eine Referenzspannungsquelle 45 vorgesehen. Zu der in der Fig. 6a gezeigten Variante korrespondiert die Schaltung gemäß Fig. 5a, in der ein mit dem Analog/Digital-Wandler 33 verbundener NTC-Widerstand vorgesehen ist, der in der Fig. 6a fehlt.

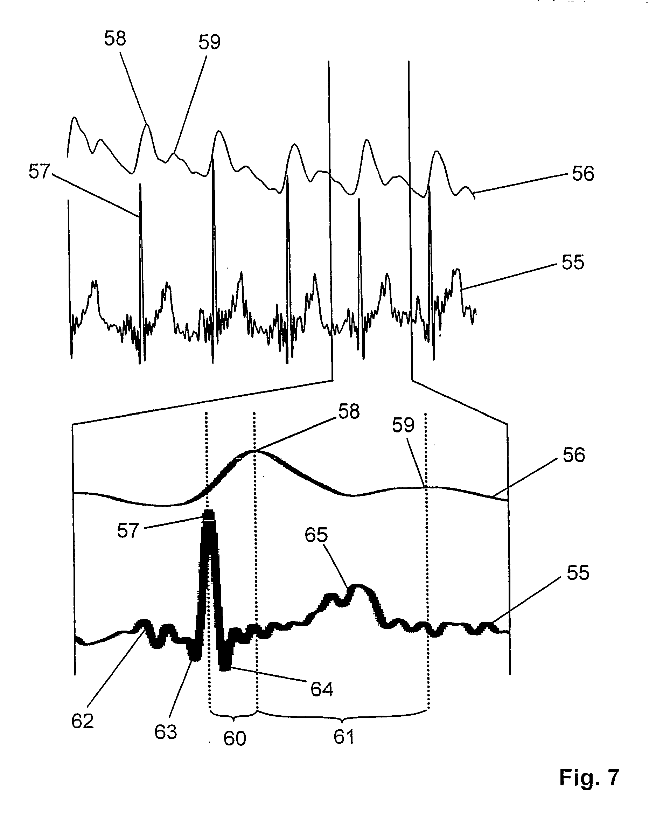

Die Fig. 7 zeigt oben ein EKG-Signal 55 zusammen mit einem Volumenpuls-Signal 56 als Funktion der Zeit. Die Signale 55 und 56 werden gleichzeitig mit dem erfindungsgemäßen mobilen Diagnosegerät aufgenommen. In dem EKG-Signal 55 ist eine Mehrzahl von R-Zacken 57 zu sehen. Jede der R-Zacken 57 zeigt einen ventrikulären Herzschlag an. In dem Volumenpuls-Signal sind Hauptmaxima 58 und Nebenmaxima 59 zu sehen. Dies zeigt die Dikrotie, d. h. die Doppelgipfeligkeit des Blutdruckverlaufs in den von der Pulsoximetrie-Einheit 2 erfassten Gefäßen. Das untere Diagramm der Fig. 7 zeigt einen Zeitausschnitt des EKG-Signals 55 und des Volumenpuls-Signals 56 in vergrößerter Darstellung. In dieser Darstellung ist zu erkennen, dass zwischen der R-Zacke 57 und dem Hauptmaximum 58 des Volumenpuls-Signals 56 eine Zeitdifferenz 60 besteht. Dieser zeitliche Abstand wird gemäß der Erfindung ermittelt. Der Zeitabstand 60 hängt von der Pulswellengeschwindigkeit ab. Ein großer Zeitabstand deutet auf eine geringe Pulswellengeschwindigkeit hin. Ein kurzer Zeitabstand bedeutet eine hohe Pulswellengeschwindigkeit. Eine deutlich erhöhte Pulswellengeschwindigkeit ist wiederum ein Indiz für das Vorliegen einer Arteriosklerose, da die Pulswellengeschwindigkeit von der Elastizität der Blutgefäße abhängt. Zur Bestimmung der Pulswellengeschwindigkeit kann ebenso gut der zeitliche Abstand zwischen der R-Zacke 57 und einem der darauf folgenden Minima des Volumenpuls-Signals 56 herangezogen werden. Dieses Vorgehen kann aus Gründen der Signalverarbeitung Vorteile haben. Das erfindungsgemäße Diagnosegerät wertet außerdem den Zeitabstand 61 zwischen dem Hauptmaximum 58 und dem Nebenmaximum 59 des Volumenpuls-Signals sowie die relativen Intensitäten des Hauptmaximums 58 und des Nebenmaximums 59 aus. Aus diesen Parametern kann mit hoher Zuverlässigkeit auf den kardiovaskulären Zustand, insbesondere hinsichtlich der Elastizität der Blutgefäße, geschlossen werden. In dem EKG-Signal 55 sind außerdem die P-Welle 62, die Minima Q und S 63 bzw. 64 sowie die T-Welle 65 zu sehen. Die Zeitabstände zwischen diesen charakteristischen Merkmalen des EKG-Signals 55 werden mittels des erfindungsgemäßen Diagnosegerätes automatisch ausgewertet.

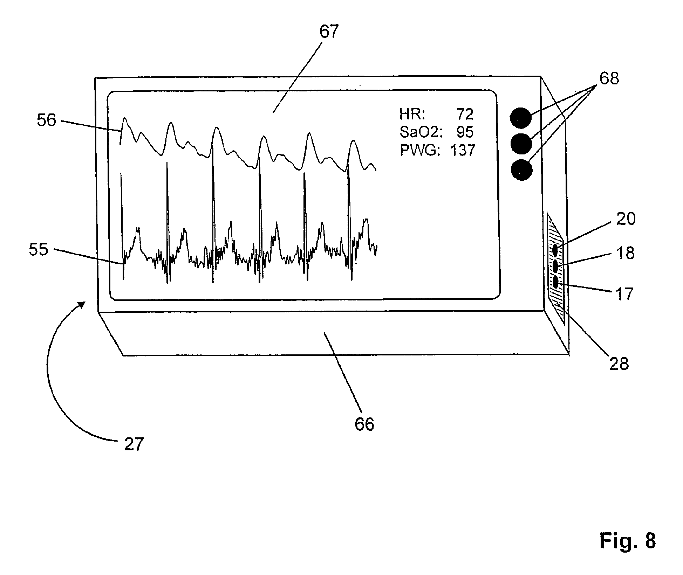

Die Fig. 8 zeigt eine Ansicht des erfindungsgemäßen Diagnosegerätes. Dieses besteht aus einem im Wesentlichen quaderförmigen Gehäuse 66, an dessen Oberseite eine LCD-Anzeige 67 angeordnet ist. Diese bildet die Anzeigeeinheit 7 des Diagnosegerätes. Auf der LCD-Anzeige 67 wird das EKG-Signal 55 sowie das Volumenpuls-Signal 56 als Funktion der Zeit graphisch angezeigt. Gleichzeitig werden die ventrikuläre Herzfrequenz HR, die Sauerstoffsättigung des Blutes $SaO_2$ sowie die Pulswellengeschwindigkeit PWG angezeigt. An der Außenseite des Gehäuses 66 sind die beiden EKG-Elektroden 27 und 28 angeordnet, und zwar in der Weise, dass der Patient mit einer Hand die Elektrode 27 und mit der anderen Hand die Elektrode 28 berühren kann. In die Elektrode 28 sind die Leuchtdioden 17 und 18 sowie der Lichtsensor, d. h. die Photodiode 20 integriert. Somit erfasst die Pulsoximetrie-Einheit des Diagnosegerätes das Volumenpuls-Signal an der Hand, mit der der Patient die Elektrode 28 berührt. An der Vorderseite des Gehäuses 66 sind Schalter 68 zur Bedienung des Diagnosegerätes angeordnet.

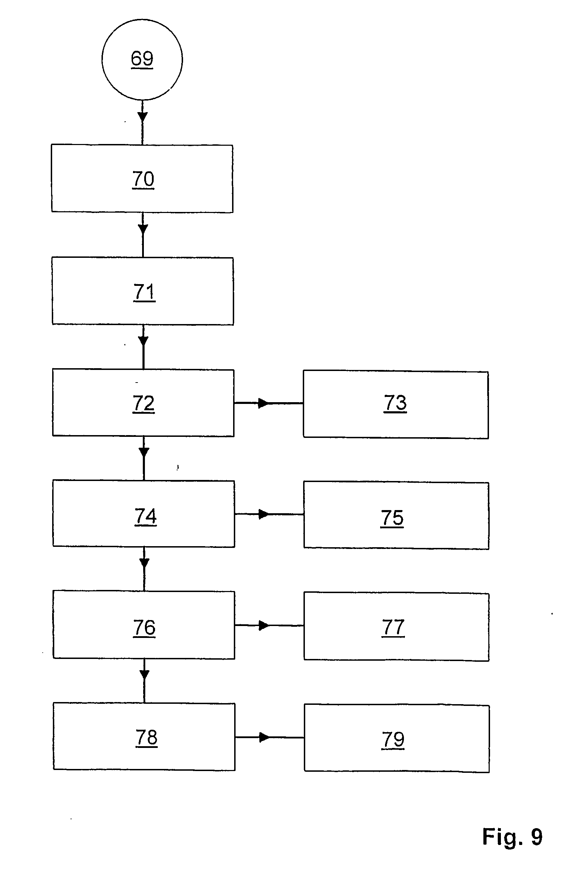

Anhand der Fig. 9 wird im Folgenden der durch die Programmsteuerung des Diagnosegerätes implementierte Algorithmus zur Auswertung des EKG-Signals 55 erläutert. Als Eingabedaten 69 erhält der Algorithmus das digitale EKG-Signal. Dieses wird zunächst einer Tiefpassfilterung 70 unterzogen, um das Signalrauschen zu reduzieren. Im nächsten Verfahrensschritt 71 wird die erste zeitliche Ableitung des EKG-Signals gebildet. Im Verfahrensschritt 72 werden sodann die R-Zacken anhand von Nulldurchgängen des abgeleiteten Signals von positiven zu negativen Werten automatisch erkannt. Aus den Zeitabständen zwischen den R-Zacken wird im Verfahrensschritt 63 die ventrikuläre Herzfrequenz bestimmt. Zwei unmittelbar aufeinander folgend ermittelte Werte der Herzfrequenz werden miteinander verglichen und auf ihre Ähnlichkeit hin überprüft. Wenn die Werte innerhalb gewisser Grenzen gleich sind, wird die ventrikuläre Herzfrequenz mittels der Anzeigeeinheit 7 des Diagnosegerätes angezeigt. Nach beispielsweise zehn aufeinander folgend ermittelten Werten der Herzfrequenz wird ein Qualitätsparameter berechnet. Dieser Qualitätsparameter Q berechnet sich nach der folgenden Formel: $Q = (N_s -1) / N_t$

Dabei ist $N_s$ die Zahl der als ähnlich befundenen Herzfrequenzwerte und $N_t$ die Gesamtzahl der ermittelten Herzfrequenzwerte. $N_t$ ist also beispielsweise gleich zehn. Der Qualitätsparameter Q kann ebenfalls mittels der Anzeigeeinheit 7 des Diagnosegerätes angezeigt werden. Ein kleiner Wert von Q deutet auf einen arrhythmischen Herzschlag hin. Im Verfahrensschritt 74 werden in dem EKG-Signal die Minima unmittelbar vor und nach den R-Zacken bestimmt. Daraus können dann in dem Verfahrensschritt 75 sämtliche Parameter des QRS-Komplexes des EKG-Signals berechnet werden. Das erste Maximum nach jeder R-Zacke wird im Verfahrensschritt 76 ermittelt. Dieses Maximum stellt die T-Welle des EKG-Signals dar. Im Verfahrensschritt 77 wird das QT-Intervall, d. h. der Zeitabstand zwischen dem QRS-Komplex und der T-Welle berechnet. Schließlich wird im Verfahrensschritt 78 ein Maximum zwischen der im Verfahrensschritt 76 detektierten T-Welle und der nächsten R-Zacke ermittelt. Hieraus kann dann wiederum das PR-Intervall, d. h. der Zeitabstand zwischen der P-Welle und der R-Zacke im Verfahrensschritt 79 berechnet werden.

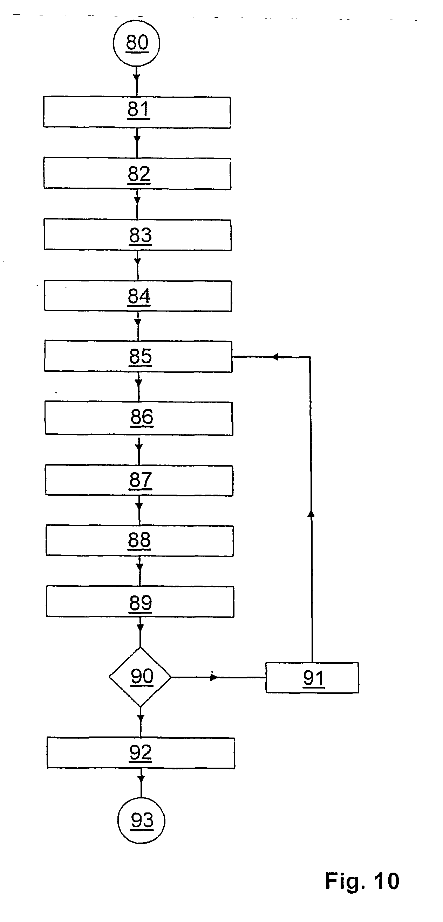

Die Fig. 10 veranschaulicht den Algorithmus des erfindungsgemäßen Diagnosegerätes zur Ermittlung der plethysmographischen Herzfrequenz. Der Algorithmus geht aus von dem digitalen Volumenpuls-Signal 80. Dieses wird in einem Verfahrensschritt 81 zunächst Tiefpass-gefiltert, um Signalrauschen zu reduzieren, danach erfolgt in einem Verfahrensschritt 82 die Berechnung der ersten zeitlichen Ableitung des Volumenpuls-Signals. Die erste zeitliche Ableitung wird erneut zur Reduzierung von Signalrauschen im Verfahrensschritt 83 Tiefpass-gefiltert. In Verfahrensschritt 84 wird ein Schwellenwert festgesetzt. Dieser kann beispielsweise dem absoluten Minimum des Volumenpuls-Signals während eines vorgebbaren Zeitintervalls von beispielsweise 10 s entsprechen. Dann werden im Verfahrensschritt 85 Durchgänge des Volumenpuls-Signals durch die zuvor festgesetzte Schwelle ermittelt. Im Verfahrensschritt 86 werden lokale Minima in dem abgeleiteten Signal zwischen den Durchgängen durch den zuvor bestimmten Schwellenwert bestimmt. Der Zeitabstand zwischen den lokalen Minima in dem abgeleiteten Signal entspricht dem Zeitabstand zwischen zwei aufeinander folgenden Wendepunkten in dem ursprünglichen Volumenpuls-Signal. Daher kann in dem Verfahrensschritt 87 aus dem Zeitabstand zwischen den lokalen Minima die plethysmographische Herzfrequenz berechnet werden. Ähnlich wie bei der Bestimmung der ventrikulären Herzfrequenz werden im Verfahrensschritt 88 die zeitlich aufeinander folgend ermittelten Werte der plethysmographischen Herzfrequenz auf Ähnlichkeit überprüft. Im Verfahrensschritt 89 wird für eine vorgebbare Zahl von aufeinander folgend ermittelten Herzfrequenzwerten ein Qualitätsparameter berechnet. Auch dies erfolgt ähnlich wie bei der Ermittlung der ventrikulären Herzfrequenz. Der Qualitätsparameter wird im Verfahrensschritt 90 bewertet und ggf. wird im Verfahrensschritt 91 der Schwellenwert erhöht, und das Verfahren wird beginnend mit dem Verfahrensschritt 85 erneut durchlaufen. Die Wiederholung endet, wenn der Qualitätsparameter einen maximalen Wert erreicht hat. Dann wird im Verfahrensschritt 92 aus den einzelnen Herzfrequenzwerten ein gemittelter plethysmographischer Herzfrequenzwert berechnet. Dieser wird dann zusammen mit dem maximalen Qualitätsparameter angezeigt. Damit endet der Algorithmus im Verfahrensschritt 93.

**Patentansprüche**

1. Mobiles Diagnosegerät mit einer EKG-Einheit (1) zur Erfassung eines EKG-Signals (55), wobei die EKG-Einheit (1) mit zwei oder mehr EKG-Elektroden (27, 28) zur Ableitung von elektrischen Signalen von dem Körper eines Patienten verbunden oder verbindbar ist, einer Pulsoximetrie-Einheit (2) zur gleichzeitigen Erfassung eines Volumenpuls-Signals (56), wobei die Pulsoximetrie-Einheit (2) wenigstens eine Lichtquelle (17, 18) und wenigstens einen Lichtsensor (20) zur optischen Messung der Blutperfusion im Gefäßsystem des Körpergewebes des Patienten aufweist, und mit einer programmgesteuerten Auswertungseinheit (4) zur Auswertung des EKG-Signals (55) und des Volumenpuls-Signals (56), wobei die Auswertungseinheit (4) eingerichtet ist

   - zur automatischen Erkennung von R-Zacken (57) in dem EKG-Signal (55),
   - zur automatischen Erkennung von Extrema (58) in dem Volumenpuls-Signal (56),
   - und zur Ermittlung des zeitlichen Abstands (60) zwischen einer R-Zacke (57) in dem EKG-Signal (55) und einem darauf folgenden Extremum (58) in dem Volumenpuls-Signal (56),

   **gekennzeichnet durch**
   ein Gehäuse (66), das die EKG-Einheit (1), die Pulsoximetrie-Einheit (2) und die Auswertungseinheit (4) aufnimmt, wobei die EKG-Elektroden (27, 28) ohne Kabelverbindung an das Diagnosegerät angeschlossen sind, und wobei die EKG-Elektroden (27, 28) sowie die Lichtquelle (17, 18) und der Lichtsensor (20) der Pulsoximetrie-Einheit (2) derart an der Außenseite des Gehäuses (66) angeordnet sind, dass der Patient mit einer Hand eine erste EKG-Elektrode (27) und mit der anderen Hand eine zweite EKG-Elektrode (28) und gleichzeitig den Lichtsensor (20) berühren kann.

2. Diagnosegerät nach Anspruch 1, **dadurch gekennzeichnet, dass** die Auswertungseinheit (4) weiterhin eingerichtet ist

   - zur Ermittlung der Blutsauerstoffsättigung aus dem Volumenpuls-Signal (56),
   - zur Ermittlung der ventrikulären Herzfrequenz aus dem EKG-Signal (55),
   - und/oder zur Ermittlung der plethysmographischen Herzfrequenz aus dem Volumenpuls-Signal (56).

3. Diagnosegerät nach Anspruch 1 oder 2, **dadurch**

**gekennzeichnet, dass** die Auswertungseinheit (4) eingerichtet ist zur automatischen Erkennung von Haupt- und Nebenmaxima (58, 59) in dem Volumenpuls-Signal (56), zur Ermittlung der Amplituden der Haupt- und Nebenmaxima (58 59) und zur Ermittlung des Zeitabstands (61) zwischen Haupt- und Nebenmaxima (58, 59).

4. Diagnosegerät nach einem der Ansprüche 1 bis 3, **gekennzeichnet durch** Sensoren (53) zur Messung der Körpertemperatur des Patienten, der Umgebungstemperatur und/oder der Luftfeuchtigkeit.

5. Diagnosegerät nach einem der Ansprüche 1 bis 4, **gekennzeichnet durch** eine Speichereinheit (6) zur Speicherung der mittels der Auswertungseinheit (4) bei einer Messung ermittelten Parameter unter gleichzeitiger Speicherung des Datums und/oder der Uhrzeit der Messung.

6. Diagnosegerät nach einem der Ansprüche 1 bis 5, **gekennzeichnet durch** eine Diagnoseeinheit (5), die eingerichtet ist zur Bestimmung des Status des Herz-Kreislauf-Systems aus den mittels der Auswertungseinheit (4) ermittelten Parametern.

7. Diagnosegerät nach den Ansprüchen 5 und 6, **dadurch gekennzeichnet, dass** die Diagnoseeinheit (5) weiterhin dazu eingerichtet ist, aus der Veränderung der mittels der Speichereinheit (6) gespeicherten Parameter Trends hinsichtlich der Veränderung des Status des Herz-Kreislauf-Systems des Patienten zu bestimmen.

8. Diagnosegerät nach einem der Ansprüche 6 und 7, **dadurch gekennzeichnet, dass** die Diagnoseeinheit (5) eingerichtet ist zur Berechnung eines Elastizitätsparameters aus dem zeitlichen Abstand (60) zwischen einer R-Zacke (57) in dem EKG-Signal (55) und einem darauf folgenden Extremum (58) in dem Volumenpuls-Signal (56), wobei der Elastizitätsparameter ein Maß für die Elastizität der Blutgefäße des Patienten ist.

9. Diagnosegerät nach einem der Ansprüche 1 bis 8, **gekennzeichnet durch** eine Schnittstelle (8) zur Verbindung des Diagnosegerätes mit einem Computer.

10. Diagnosegerät nach einem der Ansprüche 1 bis 9, **gekennzeichnet durch** eine Anzeigeeinheit (7) zur Anzeige des EKG-Signals (55), des Volumenpuls-Signals (56) und der mittels der Auswertungseinheit (4) ermittelten Parameter.

11. Diagnosegerät nach einem der Ansprüche 1 bis 10, **gekennzeichnet durch** ein Computerprogramm das Instruktionen umfasst

- zur automatischen Erkennung von R-Zacken (57) in einem EKG-Signal (55),
- zur automatischen Erkennung von Extrema (58) in einem Volumenpuls-Signal (56),
- zur Ermittlung des zeitlichen Abstands (60) zwischen einer R-Zacke (57) in dem EKG-Signal (55) und einem darauf folgenden Extremum (58) in dem Volumenpuls-Signal (56),
- und zur Berechnung der Pulswellengeschwindigkeit (PWG) aus dem zeitlichen Abstand (60) zwischen einer R-Zacke (57) in dem EKG-Signal (55) und einem darauf folgenden Extremum (58) in dem Volumenpuls-Signal (56),
- und zur automatischen Erkennung von Haupt- und Nebenmaxima (58, 59) in dem Volumenpuls-Signal (56), zur Ermittlung des Zeitabstands (61) zwischen Haupt- und Nebenmaxima (58, 59),
- und zur Berechnung einer zweiten Pulswellengeschwindigkeit aus dem zeitlichen Abstand zwischen Haupt- und Nebenmaxima (58, 59).

12. Verfahren zur Erfassung und Auswertung von Herz-Kreislauf-Parametern eines Patienten unter Verwendung eines Diagnosegerätes nach einem der Ansprüche 1 bis 10, wobei

- ein EKG-Signal (55) mittels einer EKG-Einheit (1) erfasst wird, die mit zwei oder mehr EKG-Elektroden (27, 28) zur Ableitung von elektrischen Signalen von dem Körper des Patienten verbunden ist,
- gleichzeitig ein Volumenpuls-Signal (56) mittels einer Pulsoximetrie-Einheit (2) erfasst wird, die wenigstens eine Lichtquelle (17, 18) und wenigstens einen Lichtsensor (20) zur optischen Messung der Blutperfusion im Gefäßsystem des Körpergewebes des Patienten aufweist,
- und das EKG-Signal (55) und das Volumenpuls-Signal (56) mittels einer programmgesteuerten Auswertungseinheit (4) ausgewertet werden, wobei mittels der Auswertungseinheit (4)
- R-Zacken (57) in dem EKG-Signal (55) automatisch erkannt werden,
- Extrema (58) in dem Volumenpuls-Signal (56), automatisch erkannt werden,
- und der zeitliche Abstand (60) zwischen einer R-Zacke (57) in dem EKG-Signal (55) und dem darauf folgenden Extremum (58) in dem Volumenpuls-Signal (56) ermittelt wird,
- und mittels der Auswertungseinheit (4) automatisch Haupt- und Nebenmaxima (58, 59) in dem Volumenpuls-Signal (56) erkannt werden, und die Amplituden der Haupt- und Nebenmaxima (58 59) und der Zeitabstand (61) zwischen Haupt- und Nebenmaxima (58, 59) ermittelt werden.

**Claims**

1. A mobile diagnostic device comprising an ECG unit (1) for recording an ECG signal (55), wherein the ECG unit (1) is connected or connectable to two or more ECG electrodes (27, 28) for deriving electrical signals from the body of a patient, a pulsoximetry unit (2) for simultaneously recording a volume pulse signal (56), wherein the pulsoximetry unit (2) has at least one light source (17, 18) and at least one light sensor (20) for optical measurement of blood perfusion in the vascular system of the body tissue of the patient, and a program-controlled evaluation unit (4) for evaluation of the ECG signal (55) and the volume pulse signal (56), wherein the evaluation unit (4) is adapted

- to automatically detect R peaks (57) in the ECG signal (55),
- to automatically detect extremes (58) in the volume pulse signal (56),
- and to ascertain the time spacing (60) between an R peak (57) in the ECG signal (56) and a subsequent extreme (58) in the volume pulse signal (56),

**characterised by** a housing (66) which accommodates the ECG unit (1), the pulsoximetry unit (2) and the evaluation unit (4), wherein the ECG electrodes (27, 28) are connected to the diagnostic device without a cable connection and wherein the ECG electrodes (27, 28) as well as the light source (17, 18) and the light sensor (20) of the pulsoximetry unit (2) are arranged at the outside of the housing (66) in such a way that the patient can touch a first ECG electrode (27) with one hand and a second ECG electrode (28) and at the same time the light sensor (20) with the other hand.

2. A diagnostic device according to claim 1 **characterised in that** the evaluation unit (4) is further adapted

- to ascertain the blood oxygen saturation from the volume pulse signal (56),
- to ascertain the ventricular heart rate from the ECG signal (55),
- and/or to ascertain the plethysmographic heart rate from the volume pulse signal (56).

3. A diagnostic device according to claim 1 or claim 2 **characterised in that** the evaluation unit (4) is adapted to automatically detect main and secondary maxima (58, 59) in the volume pulse signal (56), to ascertain the amplitudes of the main and secondary maxima (58, 59) and to ascertain the time spacing (61) between main and secondary maxima (58, 59).

4. A diagnostic device according to one of claims 1 to 3 **characterised by** sensors (53) for measuring the body temperature of the patient, the ambient temperature and/or air humidity.

5. A diagnostic device according to one of claims 1 to 4 **characterised by** a memory unit (8) for storing the parameters ascertained by means of the evaluation unit (4) in a measurement procedure with simultaneous storage of the date and/or time of the measurement.

6. A diagnostic device according to one of claims 1 to 5 **characterised by** a diagnostic unit (5) adapted to determine the status of the cardiovascular system from the parameters ascertained by means of the evaluation unit (4).

7. A diagnostic device according to claims 5 and 6 **characterised in that** the diagnostic unit (5) is further adapted to determine trends in respect of the change in the status of the cardiovascular system of the patient from the change in the parameters stored by means of the memory unit (6).

8. A diagnostic device according to one of claims 6 and 7 **characterised in that** the diagnostic unit (5) is adapted to compute an elasticity parameter from the time spacing (60) between an R peak (57) in the ECG signal (55) and a subsequent extreme (58) in the volume pulse signal (56), wherein the elasticity parameter is a measurement in respect of the elasticity of the blood vessels of the patient.

9. A diagnostic device according to one of claims 1 to 8 **characterised by** an interface (8) for connecting the diagnostic device to a computer.

10. A diagnostic device according to one of claims 1 to 9 **characterised by** a display unit (7) for displaying the ECG signal (55), the volume pulse signal (56) and the parameters ascertained by means of the evaluation unit (4).

11. A diagnostic device according to one of claims 1 to 10 **characterised by** a computer program which includes instructions

    - for automatically detecting R peaks (57) in an ECG signal (55),
    - for automatically detecting extremes (58) in a volume pulse signal (56),
    - for ascertaining the time spacing (60) between an R peak (57) in the ECG signal (55) and a subsequent extreme (58) in the volume pulse signal (56),
    - for computing the pulse wave velocity (PWG) from the time spacing (60) between an R peak (57) in the ECG signal (55) and a subsequent extreme (58) in the volume pulse signal (56),
    - for automatically detecting main and secondary maxima (58, 59) in the volume pulse signal (56), for ascertaining the time spacing (61) between main and secondary maxima (58, 59),
    - and for computing a second pulse wave velocity from the time spacing between main and secondary maxima (58, 59).

12. A method of recording and evaluating cardiovascular parameters of a patient using a diagnostic device according to one of claims 1 to 10, wherein

    - an ECG signal (55) is recorded by means of an ECG unit (1) which is connected to two or more ECG electrodes (27, 28) for deriving electrical signals from the body of the patient,
    - at the same time a volume pulse signal (56) is recorded by means of a pulsoximetry unit (2) which has at least one light source (17, 18) and at least one light sensor (20) for optical measurement of the blood perfusion in the vascular system of the body tissue of the patient,
    - and the ECG signal (55) and the volume pulse signal (56) are evaluated by means of a program-controlled evaluation unit (4), wherein by means of the evaluation unit (4)
    - R peaks (57) in the ECG signal (55) are automatically detected,
    - extremes (58) in the volume pulse signal (56) are automatically detected,
    - and the time spacing (60) between an R peak (57) in the ECG signal (55) and the subsequent extreme (58) in the volume pulse signal (56) is ascertained,
    - and by means of the evaluation unit (4) main and secondary maxima (58, 59) in the volume pulse signal (56) are automatically detected, and the amplitudes of the main and secondary maxima (58, 59) and the time spacing (61) between main and secondary maxima (58, 59) are ascertained.

## Revendications

1. Appareil de diagnostic mobile avec une unité ECG (1) pour la détection d'un signal ECG (55), où l'unité ECG (1) est reliée ou peut être reliée à deux électrodes ECG (27,28) ou plus pour la dérivation de signaux électriques du corps d'un patient, une unité formant pulsoxymètre (2) pour la détection simultanée d'un signal de pouls volumique (56), où l'unité formant pulsoxymètre (2) présente au moins une source de lumière (17,18) et au moins un capteur de lumière (20) pour la mesure optique de la perfusion de sang dans le système vasculaire du tissu corporel du patient, et avec une unité d'évaluation (4) commandée par programme pour l'évaluation du signal ECG (55) et du signal de pouls volumique (56), où l'unité d'évaluation (4) est conçue

- pour la détection automatique d'ondes R (57) dans le signal ECG (55),
- pour la détection automatique d'extrêmes (58) dans le signal de pouls volumique (56),
- et pour la détermination de l'écart temporel (60) entre une onde R (57) dans le signal ECG (55) et un extrême (58) faisant suite à celle-ci dans le signal de pouls volumique (56),

**caractérisé par** un boîtier (66) qui reçoit l'unité ECG (1), l'unité formant pulsoximètre (2) et l'unité d'évaluation (4), où les électrodes ECG (27,28) sont connectées sans liaison par câble à l'appareil de diagnostic, et où les électrodes ECG (27,27) ainsi que la source de lumière (17,18) et le capteur de lumière (20) de l'unité formant pulsoxymètre (2) sont disposées de telle sorte au côté extérieur du boîtier (66) que le patient peut toucher avec une main une première électrode ECG (27) et avec l'autre main une seconde électrode ECG (28) et en même temps le capteur de lumière (20).

2. Appareil de diagnostic selon la revendication 1, **caractérisé en ce que** l'unité d'évaluation (4) est conçue en outre

- pour la détermination de la saturation de l'oxygène du sang à partir du signal de pouls volumique (56),
- pour la détermination de la fréquence cardiaque ventriculaire à partir du signal ECG (55),
- et/ou pour la détermination de la fréquence cardiaque pléthysmographe à partir du signal de pouls volumique (56).

3. Appareil de diagnostic selon la revendication 1 ou 2, **caractérisé en ce que** l'unité d'évaluation (4) est conçue pour la détection automatique de maxima principal et secondaire (58,59) dans le signal de pouls volumique (56), pour déterminer les amplitudes des maximas principal et secondaire (58,59) et pour déterminer l'écart de temps (61) entre les maximas principal et secondaire (58,59).

4. Appareil de diagnostic selon l'une des revendications 1 à 3, **caractérisé par** des capteurs (53) pour la mesure de la température du corps du patient, de la température ambiante et/ou de l'humidité de l'air.

5. Appareil de diagnostic selon l'une des revendications 1 à 4, **caractérisé par** une unité de stockage (6) pour stocker les paramètres déterminés au moyen de l'unité d'évaluation (4) lors d'une mesure en stockant simultanément la date et/ou l'heure de la mesure.

6. Appareil de diagnostic selon l'une des revendications 1 à 5, **caractérisé par** une unité de diagnostic (5) qui est conçue pour la détermination du status du système cardiovasculaire à partir des paramètres déterminés au moyen de l'unité d'évaluation (4).

7. Appareil de diagnostic selon les revendications 5 et 6, **caractérisé en ce que** l'unité de diagnostic (5) est conçue en outre pour déterminer à partir de la modification des paramètres stockés au moyen de l'unité de stockage (6) des tendances concernant la modification du status du système cardiovasculaire du patient.

8. Appareil de diagnostic selon l'une des revendications 6 et 7, **caractérisé en ce que** l'unité de diagnostic (5) est conçue pour le calcul d'un paramètre d'élasticité à partir de l'écart temporel (60) entre une onde R(57) dans le signal ECG (55) et un extrême (58) faisant suite à celle-ci dans le signal de pouls volumique (56), où le paramètre d'élasticité est une mesure pour l'élasticité des vaisseaux du patient.

9. Appareil de diagnostic selon l'une des revendications 1 à 8, **caractérisé par** une interface (8) pour relier l'appareil de diagnostic à un ordinateur.

10. Appareil de diagnostic selon l'une des revendications 1 à 9, **caractérisé par** une unité d'affichage (7) pour l'affichage du signal ECG (55), du signal de pouls volumique (56) et des paramètres déterminés au moyen de l'unité d'évaluation (4).

11. Appareil de diagnostic selon l'une des revendications 1 à 10, **caractérisé par** un programme informatique qui comprend des instructions

- pour la détection automatique d'ondes R (57) dans un signal ECG (55),
- pour la détection automatique d'extrêmes (58) dans un signal de pouls volumique (56),
- pour la détermination de l'écart temporel (60) entre une onde R (57) dans le signal ECG (55) et un extrême (58) faisant suite à celle-ci dans le signal de pouls volumique (56),
- et pour le calcul de la vitesse d'ondes de pouls (PWG) à partir de l'écart temporel (60) entre une onde R (57) dans le signal ECG(55) et un extrême (58) faisant suite à celle-ci dans le signal de pouls volumique (56),
- et pour la détection automatique de maxima principal et secondaire (58,59) dans le signal de pouls volumique (56), pour la détermination de l'écart temporel (61) entre les maximas principal et secondaire (58,59),
- et pour le calcul d'une deuxième vitesse d'onde de pouls à partir de l'écart temporel entre les maximas principal et secondaire (58,59).

12. Procédé de détection et d'évaluation de paramètres cardiovasculaires d'un patient en utilisant un appareil de diagnostic selon l'une des revendications 1 à 10, où

   - un signal ECG (55) est détecté au moyen d'une unité ECG (1), qui est reliée à deux électrodes ECG ou plus (27,28) pour la dérivation de signaux électriques du corps du patient,
   - en même temps un signal de pouls volumique (56) est détecté au moyen d'une unité formant pulsoxymètre (2), qui présente au moins une source de lumière (17,18) et au moins un capteur de lumière (20) pour la mesure optique de la perfusion du sang dans le système vasculaire du tissu corporel du patient,
   - et le signal ECG (55) et le signal de pouls volumique (56) sont évalués au moyen d'une unité d'évaluation (4) commandée par programme, où au moyen de l'unité d'évaluation (4)
   - des ondes R (57) dans le signal ECG (55) sont détectées automatiquement,
   - des extrêmes (58) dans le signal de pouls volumique (56) sont détectés automatiquement,
   - et l'écart temporel (60) entre une onde R (57) dans le signal ECG (55) et l'extrême (58) faisant suite à celle-ci est déterminé dans le signal de pouls volumique (56),
   - et en ce que sont détectés au moyen de l'unité d'évaluation (4) automatiquement des maximas principal et secondaire (58,59) dans le signal de pouls volumique (56), et les amplitudes des maximas principal et secondaire (58,59) et l'écart temporel (61) entre les maximas principal et secondaire (58,59) sont déterminés.

**Fig. 1**

**Fig. 2**

Fig. 3

Fig. 4

**Fig. 5**

**Fig. 5a**

Fig. 6

**Fig. 6a**

Fig. 7

67

68

56

HR:      72
SaO2:   95
PWG:  137

20

18

17

55

28

27

66

**Fig. 8**

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4960126 A
- US 20020087087 A1
- US 5309916 A